# EUROPÄISCHE PATENTANMELDUNG

(11) **EP 1 974 723 A2**
(43) Veröffentlichungstag der Anmeldung: **01.10.2008**
(21) Anmeldenummer: 08011237.8
(22) Anmeldetag: 12.11.2004
(51) Int. Cl.: A61K 9/14, C07K 16/28, A61P 35/00

(54) **Feste Formen von anti-EGFR-Antikörpern**

(30) Priorität: 29.11.2003 DE 10355904
(62) Teilanmeldung aus: 04797849.9
(71) Anmelder: Merck Patent GmbH, 64293 Darmstadt (DE)
(72) Erfinder: Matheus, Susanne, 54347 Neumagen-Dhron (DE); Mahler, Hans-Christian, Dr., 65205 Wiesbaden (DE)

(57) **Zusammenfassung**

Die Erfindung betrifft feste Formen von Antikörpern gegen den EGF-Rezeptor, insbesondere Präzipitate und Kristalle von monoklonalen Antikörpern gegen den EGF-Rezeptor, besonders bevorzugt von Mab C225 (Cetuximab) und Mab h425 (EMD 72000), welche durch Auflösen oder Suspendieren in wässrigem Medium zu biologisch aktivem Antikörperprotein führen, erhältlich durch Ausfällung des in wässrigem Medium gelösten oder suspendierten Antikörpers und/oder einer seiner Varianten und/oder Fragmente mittels eines Präzipitationsreagenzes. Die Erfindung betrifft weiterhin pharmazeutische Zubereitungen enthaltend wenigstens eine feste Form obengenannter Antikörper in präzipitierter nicht-kristalliner, präzipitierter kristalliner oder in löslicher oder suspendierter Form, sowie gegebenenfalls Träger- und/oder Hilfsstoffe und/oder weitere pharmazeutische Wirkstoffe, sowie ein Verfahren zur Herstellung erfindungs-gernäßer fester Formen von anti-EGFR-Antikörpem.

## Beschreibung

### Hintergrund der Erfindung

Die Erfindung betrifft feste Formen von Antikörpern gegen den EGF-Rezeptor (EGFR), insbesondere Präzipitate und Kristalle von monoklonalen Antikörpern gegen den EGF-Rezeptor, besonders bevorzugt von Mab C225 (Cetuximab) und Mab h425 (EMD 72000), welche durch Auflösen oder Suspendieren in wässrigem oder nicht-wäßrigem Medium zu biologisch aktivem Antikörperprotein führen, erhältlich durch Ausfällung, des in wässrigem Medium gelösten oder suspendierten Antikörpers und/oder einer seiner Varianten und/oder Fragmente mittels eines Präzipitationsreagenz. Die Erfindung betrifft weiterhin pharmazeutische Zubereitungen enthaltend wenigstens eine feste Form obengenannter Antikörper in präzipitierter nicht-kristalliner, präzipitierter kristalliner oder in gelöster oder suspendierter Form, sowie gegebenenfalls Träger- und/oder Hilfsstoffe und/oder weitere pharmazeutische Wirkstoffe, sowie ein Verfahren zur Herstellung erfindungsgemäßer fester Formen von anti-EGFR-Antikörpem.

Fortschritte im Bereich der Biotechnologie machten es im Laufe der letzten 10 Jahre möglich eine Reihe von Proteinen zur pharmazeutischen Anwendung mittels rekombinanter DNA-Techniken herzustellen. Proteinarzneimittel wie monoklonale Antikörper finden ihre Anwendung beispielsweise in der Tumortherapie, z.B. zur spezifischen Immuntherapie oder Tumorvakzinierung. Therapeutische Proteine sind größer und komplexer als herkömmliche organische und anorganische Wirkstoffe und sie weisen komplexe dreidimensionale Strukturen und zahlreiche furtktionelle Gruppen auf, die die biologische Aktivität des Proteins bedingen oder auch unerwünschte Wirkungen hervorrufen können. Proteinarzneimittel sind während Herstellung, Lagerung und Transport zahlreichen exogenen Einflüssen ausgesetzt, die sich stabilitätsmindernd auf den Proteinwirkstoff auswirken können. Es ist daher erforderlich, die Ursachen und Mechanismen der speziellen Abbaureaktionen genau zu studieren, um z.B. durch Zusatz bestimmter stabilisierender Hilfsstoffe das Protein stabilisieren zu können (siehe z.B. Manning M.C., Patel K., & Borchardt R.T. (1989) Stability of protein pharmaceuticals. Pharm. Res. 6, 903-918).

Aus der Literatur sind zahlreiche Formulierungen therapeutischer Proteine bekannt. Die Anforderungen an die Zusammensetzung einer pharmazeutischen Zubereitung von Proteinwirkstoffen können jedoch sehr unterschiedlich sein und im Allgemeinen ist es aufgrund spezifischer physikochemischer Eigenschaften und Abbaureaktionen der unterschiedlichen Proteine nicht möglich, bereits etablierte Proteinformulierungen auf neue Protein-Wirkstoffe zu übertragen. Deshalb sind geeignete pharmazeutische Formulierungen und stabile Formen dieser neuartigen Wirkstoffe noch immer eine große Herausforderung.

Chemische Instabilitäten zeichnen sich durch kovalente Modifikationen des Proteins aus. Die Primärstruktur des Proteins ändert sich durch Bruch, Neuaufbau oder Umbildung chemischer Bindungen. Die neu entstandene Substanz unterscheidet sich in der Regel in der biologischen Aktivität komplett von dem ursprünglichen, nativen Protein. Physikalische Instabilitäten verändern die räumliche Anordnung des Moleküls (die Sekundär-, Tertiär- und Quartärstruktur), ohne kovalente Bindungen zu zerstören. Man kann sie in Denaturierung, Assoziation, Aggregation, Präzipitation oder Adsorption unterteilen. Physikalische Instabilitäten sind insbesondere bei größeren Proteinen ein häufiges Phänomen. Präzipitate sind das makroskopisch sichtbare Äquivalent von Aggregaten und entstehen mechanistisch durch Cluster von Aggregaten oder Assoziaten. Durch Überschreitung der Löslichkeitsgrenze und Ausfällung (Präzipitation) werden die Flocken ab einem Durchmesser von ca. 10 µm durch ein Lichtmikroskop und ab ca. 50 µm visuell sichtbar. Die Proteinaggregation kann ein reversibler oder irreversibler Prozess sein (siehe z.B. Cleland J.L., Powell M.F. & Shire S.J. (1993) The development of stable protein formulations: A close look at protein aggregation, deamidation, and oxidation. Crit. Rev. Ther. Drug Carrier Syst. 10, 307-377).

In bisheriger Literatur wird zwar die Präzipitation von Proteinen mit Salzen, Polymeren und organischen Lösungsmitteln als Standardmethode zur Aufreinigung von Proteinen beschrieben (Scopes R.K. (1997) Separation by Precipitation. In: Protein Purification: Principles and Practice (ed Scopes R.K.), 2 edn, pp. 41-71. Springer Verlag, New York), allerdings führt die Anwendung dieser Methode besonders bei Immunglobulinen zumeist zu einer Denaturierung, zu einer damit verbundenen Aktivitätsminderung und zu schlechten quantitativen Ausbeuten, insbesondere bei Verwendung von Salzen und organischen Lösungsmitteln (Phillips A.P., Martin K.L., & Horton W.H. (1984) The choice of methods for immunoglobulin IgG purification: Yield and purity of antibody activity. Journal of Immunological Methods 74, 385-393). Bei Verwendung von Polyethylenglycol (PEG) werden dagegen bessere Ergebnisse erzielt (A. Polson, G. M. Potgieter, J. F. Largier, and G. E. F. Joubert F. J. Mears. The Fractionation of Protein Mixtures by linear Polymers of High Molecular Weight. Biochim. Biophys. Acta 82:463-475, 1964).

Proteinkristalle sind aus den Aufreinigungsverfahren (Down Stream Processing) vorzugsweise von Enzymen und zur Aufklärung der Tertiärstruktur von Proteinen mittels Röntgenstrukturanalyse bekannt (R. K. Scopes. Analysis for purity: Crystallization. In: Protein Purification: Principles and Practice, edited by R. K. Scopes, New York:Springer Verlag, 1997, p. 284-301). Hierbei kommt es zur Bildung neuer geordneter, intermolekularer Kontakte zwischen Proteinen. Es handelt sich um einen langsamen Prozess, mit reduzierter Mobilität. Die Konzentration des in Lösung befindlichen Proteins wird dabei vermindert.

In der Literatur wird zwar die Kristallisation von Proteinen mit Salzen, Polymeren und organischen Lösungsmitteln als Standardmethode zur Strukturaufklärung von Immunglobulinen beschrieben (Harris L.J., Skaletsky E., & McPherson A. (1995) Crystallization of Intact Monoclonal Antibodies. Proteins: Structure, Function, and Genetics 23, 285-289; Harris L.J., Skaletsky E., & McPherson A. (1998) Crystallographic Structure of an Intact IgG1 Monoclonal Antibody. Journal of Molecular Biology 275, 861-872; Edmundson A.B., Guddat L.W., & Andersen K.N. (1993) Crystal Structures of intact IgG antibodies. ImmunoMethods 3, 197-210), allerdings gestaltet sich die Kristallisation von intakten, beispielsweise glykosylierten Antikörpern äußerst schwierig, da die Größe des Proteins, das unterschiedliche Glykosylierungsmuster der einzelnen Antikörpermoleküle und die damit verbundenen Mikroheterogenitäten sowie die strukturelle Flexibilität des Immunoglobulins den geordneten Einbau in ein Kristallgitter erschweren oder gar verhindern (McPherson A. (1999) Crystallization of Biological Macromolecules, 1 edn. Cold Spring Harbor Laboratory Press, New York). Außerdem zeigen Antikörpermoleküle eine Tendenz zur Aggregation, was ebenfalls große Schwierigkeiten bei der Kristallisation bereitet (McPherson A. (1999) Crystallization of Biological Macromolecules, 1 edn. Cold Spring Harbor Laboratory Press, New York). Darüber hinaus macht die Gefahr der Denaturierung der Antikörper im Rahmen des Kristallisationsvorgangs die Kristallisation von therapeutischen Antikörpern für den Fachmann unattraktiv. So wurden bisher erst wenige intakte Antikörper zur Strukturaufklärung kristallisiert bzw. erst drei Antikörper in präperativem Maßstab kristallisiert. So handelt es sich bei den in der Biological Macromolecule Crystallization Database (Gilliland, G.L., Tung, M., Blakeslee, D.M. and Ladner, J. 1994. The Biological Macromolecule Crystallization Database, Version 3.0: New Features, Data, and the NASA Archive for Protein Crystal Growth Data. Acta Crystallogr. D50 408-413.) aufgelisteten, bereits kristallisierten Immunglobulinen hauptsächlich um Fab und Fc Fragmente.

In der WO02072636 werden Antikörper-Kristalle beschrieben, die jedoch in einem aufwendigen Verfahren mit Animpfen und unter Verwendung von in pharmazeutischen Formulierungen möglichst zu vermeidenden Detergenzien und z.T. toxikologisch bedenkliche Hilfsstoffe hergestellt werden. Außerdem ist in dem beschriebenen Verfahren die Partikelgröße nicht kontrollierbar. In einem Kontrollversuch (siehe Beispiel 8) konnte gezeigt werden, dass mit dem in der WO02072636 beschriebenen Verfahren sowohl aus der Proteinlösung als auch aus der Negativkontrolle (ohne Protein) die beschriebenen nadelförmigen Kristalle erhalten werden. Daraus wird deutlich, dass es sich hier vermutlich allenfalls um Proteineinschlüsse in Kristalle des Präzipitationsreagenzes handelt.

Aus obengenannten Gründen wird klar, dass sich die Kristallisation von Antikörpern für den Fachmann als äußerst schwierig darstellt und sich aus der Literatur bekannte Kristallisationsverfahren aufgrund der großen Heterogenität der verschiedenen bekannten Antikörper bezüglich Primär-, Sekundär- und Tertiärstruktur, Glykosylierung und struktureller Flexibilität nicht auf alle bekannten Antikörper übertragen lassen. Ebenso war es aus obengenannten Gründen für den Fachmann unattraktiv, Präzipitate von therapeutischen Antikörpern herzustellen, da insbesondere mit einer irreversiblen Denaturierung zu rechnen war.

Aufgabe der vorliegenden Erfindung war es daher, stabile Formen, beispielsweise Präzipitate ode Kristalle für therapeutische Proteine, insbesondere Antikörper, zu finden, so dass bei Herstellung, Lagerung, Transport und Applikation deren Wirksamkeit erhalten bleibt. Da sich wie oben erwähnt bereits etablierte Proteinformulierungen im Allgemeinen nicht auf neue Protein-Wirkstoffe übertragen lassen, war es eine weitere Aufgabe der vorliegenden Erfindung, neue stabile Formulierungen für monoklonale Antikörper gegen den EGF-Rezeptor, beispielsweise Mab C225 (Cetuximab) und Mab h425 (EMD 72000), zu finden, Formulierungen enthaltend Mab C225 (Cetuximab) oder Mab h425 (EMD 72000) sind zwar aus der WO03053465 und aus der WO03007988 bekannt. Jedoch weisen die aus der WO03053465 bekannten Formulierungen eine relativ geringe Proteinkonzentration auf und sie sind langfristig bei Raumtemperatur nicht stabil und die aus der WO03007988 bekannten Formulierungen weisen ebenfalls eine relativ geringe Proteinkonzentration auf und die Zubereitung (Lyophilisat) muss vor Anwendung noch rekonstituiert werden. Folglich war es eine weitere Aufgabe der vorliegende Erfindung, eine stabile pharmazeutische Zubereitung zu finden, die eine hohe Konzentration obengenannter Antikörper aufweist.

Der Prozess der Lyophilisierung zur Stabilisierung von Proteinformulierungen ist beispielsweise aus der WO9300807 oder WO98221 bekannt, jedoch bestehen signifikante Nachteile von lyophilisierten Zubereitungen darin, dass der Anwender das Lyophilisat vor Anwendung noch rekonstituieren muss, was eine erhebliche Fehlerquelle bei der Zubereitung vor der Anwendung darstellt. Da ein weiterer Hersteltungsprozess im Vergleich zu flüssigen Formulierungen hinzukommt, ist der Prozess hinsichtlich zusätzlichem Aufwand für Prozessentwicklung (Gewährleistung der Stabilität bei der Lyophilisierung), Herstellung (Herstellungskosten und -dauer) und z.B. Validierung ungünstig.

Somit war es Aufgabe der vorliegenden Erfindung, feste Formen und Formulierungen für obengenannte Antikörper zu finden, die eine erhöhte Stabilität gegenüber Stressbedingungen, wie erhöhter Temperatur, Luftfeuchtigkeit und/oder Scherkräften aufweisen und keine toxikologisch bedenklichen Hilfsstoffe enthalten.

### Zusammenfassung der Erfindung

Überraschenderweise wurde gefunden, dass sich feste Formen obengenannter Antikörper herstellen lassen und diese festen Formen und daraus hergestellte Formulierungen nicht die im Stand der Technik genannten Nachteile aufweisen. Die nachfolgend beschriebenen erfindungsgemäßen festen Formen von anti-EGFR-Antikörpern und/oder die daraus hergestellten Formulierungen zeichnen sich überraschenderweise durch einen oder mehrere Vorteile aus, ausgewählt unter: hohe Stabilität, kontrollierbare Partikelgröße, Protein nach Wiederauflösung oder Resuspension nativ und biologisch aktiv, hohe Reinheit, Abwesenheit pharmazeutisch bedenklicher Agenzien und damit hohe Sicherheit, gute Verträglichkeit und Möglichkeit der direkten Verwendung, geringe Aggregationstendenz und damit die Möglichkeit hochkonzentrierte Formulierungen herzustellen und bei Formulierung als Proteinsuspension eine im Vergleich zu einer Lösung geringe Viskosität.
Das nachfolgend beschriebene erfindungsgemäße Herstellungsverfahren zeichnet sich überraschenderweise durch einen oder mehrere Vorteile aus, ausgewählt unter: Einfachheit, Zeit- und Kostenersparnis, Verwendung pharmazeutisch unbedenklicher Agenzien, hohe Ausbeute. Das erfindungsgemäße Verfahren ist somit in der Durchführung vorzugsweise wesentlich einfacher, zeitsparender und kosteneffektiver als die in der Literatur beschriebenen Techniken, da nur die Zugabe eines einzelnen Präzipitationsreagenzes notwendig ist.

Zudem erfolgt die Zugabe des Präzipitationsreagenzes zu einer Lösung des Antikörpers in einem geeigneten Puffersystem, d.h. es ist keine Stabilisierung der Reaktionslösungen durch weitere Hilfsstoffe, wie z.B. Detergenzien notwendig. Die Verwendung von Detergenzien in Zubereitungen zur parenteralen Anwendung ist generell zu vermeiden bzw. zu minimieren, da von ihnen ein nicht unerhebliches toxisches und immunogenes Potential ausgeht (Sweetana S. & Akers M.J. (1996) Solubility principles and practices for parenteral drug dosage form development. PDA J. Pharm. Sci. Technol. 50, 330-342) und sie auch zur Veränderung der Sekundärstruktur von Proteinen führen können (Vermeer A.W.P. & Norde W. (2000) The influence of the binding of low molecular weight surfactants on the thermal stability and secondary structure of IgG. Colloids and Surfaces A: Physicochemical and Engineering Aspects 161, 139-150). So können die erhaltenen festen Formen von anti-EGFR-Antikörpern direkt in Arzneimitteln verwendet werden, ein weitere Reinigung zum Entfernen pharmakologisch bedenklicher Agenzien ist nicht notwendig. Die in der WO02072636 erhaltenen Kristalle müssen dagegen in einem aufwendigen Verfahren von pharmakologisch bedenklichen Agenzien, beispielsweise CHES, Imidazol, Tris, Mangan(II)-chlorid, Zink(II)-Chlorid, Kupfer(II)-sulfat, 2-Propanol, 2-Methoyl-2,4-pentandiol, von HEPES, Lithiumsulfat, Ethoxyethanol oder Detergenzien wie Polysorbat 80 oder 20 gereinigt werden bzw. es ist gar nicht möglich vorstehend genannte, bedenkliche Agenzien vollständig zu entfernen.

Überraschenderweise wurde gefunden, dass vorzugsweise stabile feste Formen erhalten werden, wenn Antikörper gegen den EGF-Rezeptor (anti-EGFR-Antikörper), bevorzugt monoklonale anti-EGFR-Antikörper, besonders bevorzugt Mab C225 (Cetuximab) oder Mab h425 (EMD 72000), mithilfe bestimmter Präzipitationsreagenzien, ausgewählt unter Polymeren, vorzugsweise Polyethylenglycol (PEG), Salzen, vorzugsweise Ammoniumsulfat oder organischen Lösungsmitteln, vorzugsweise Ethanol, oder deren Mischungen, in Gegenwart eines geeigneten Puffers, bei geeignetem pM-Wert und geeigneter Temperatur inkubiert werden.

Überraschenderweise wurde gefunden, dass die mit erfindungsgemäßen Verfahren erhaltenen festen Formen von anti-EGFR-Antikörpern, bevorzugt von monoklonalen anti-EGFR-Antikörpern, besonders bevorzugt von Mab C225 (Cetuximab) oder Mab h425 (EMD 72000) und/oder deren Varianten oder Fragmenten, vorzugsweise nach Wiederauflösung nativ sind und vorzugsweise in einer hohen Ausbeute erhalten werden.

Gegenstand der Erfindung sind deshalb feste Formen von anti-EGFR-Antikörpern und/oder deren Varianten oder Fragmenten, welche durch Auflösen oder Suspendieren in wässrigem Medium zu biologisch aktivem Antikörperprotein führen, erhältlich durch Ausfällung des in wässrigem Medium gelösten oder suspendierten Antikörpers und/oder einer seiner Varianten und/oder Fragmente mittels eines Präzipitationsreagenzes, ausgewählt unter Polymeren, vorzugsweise Polyethylenglycol (PEG), Salzen, vorzugsweise Ammoniumsulfat, oder organischen Lösungsmitteln, vorzugsweise Ethanol, oder deren Mischungen, in Gegenwart eines geeigneten Puffers, bei geeignetem pH-Wert und geeigneter Temperatur.

### Feste Formen von anti-EGFR-Antikörpern:

Unter erfindungsgemäßen festen Formen eines anti-EGFR-Antikörpers versteht man vorzugsweise Präzipitate oder Kristalle, welche durch Auflösen oder Suspendieren in wässrigem oder nicht-wäßrigem Medium zu biologisch aktivem Antikörper führen.
Erfindungsgemäße feste Formen werden durch Ausfällen des in einem wässrigen Medium gelösten oder suspendierten Antikörpers gemäß untenstehend beschriebenen Verfahren erhalten. Die erfindungsgemäßen festen Formen können im Bereich von µm und nm liegen.

### Präzipitate:

Im Rahmen der vorliegenden Erfindung versteht man unter Präzipitaten feste Formen in amorpher nicht-kristalliner Struktur oder Aggregations-und Assoziationszustände,

### Kristalle:

Im Rahmen der vorliegenden Erfindung versteht man unter Kristallen feste Formen in kristalliner Struktur.
Kristalline Strukturen lassen sich mit untenstehenden Verfahren nachweisen.

präzipitiert: Unter präzipitiert versteht man man im Rahmen der vorliegenden Erfindung erfindungsgemäße feste Formen in ausgefällter Form, d.h. je nach Verfahrenbedingungen in Form eines Präzipitats oder eines Kristalls.

präzipitiert kristallin: Unter präzipitiert kristallin oder kristallin versteht man im Rahmen der vorliegenden Erfindung erfindungsgemäße feste Formen in geordneter kristalliner Struktur.

präzipitiert nicht-kristallin: Unter präzipitiert nicht-kristallin versteht man im Rahmen der vorliegenden Erfindung erfindungsgemäße feste Formen in amorpher nicht-kristalline Strukturen oder Aggregations-und Assoziationszustände.

gelöst: Unter gelöster Form versteht man im Rahmen der vorliegenden Erfindung erfindungsgemäße feste Formen die in einer erfindungsgemäßen Lösung gelöst bzw. wiederaufgelöst vorliegen.

suspendiert: Unter suspendierter Form versteht man erfindungsgemäße feste Formen die in einer erfindungsgemäßen Lösung suspendiert bzw, resuspendiert vorliegen.

Als "wässriges Medium" im Rahmen der Erfindung werden Wasser oder Gemische von Wasser mit geeigneten inerten Lösungsmitteln und anderen in Beispiel 1 genannten Agenzien verstanden, wie beispielsweise Puffer, Stabilisatoren oder Hilfsstoffe, mit der Eigenschaft, dass erfindungsgemäße wässrige Medien allein nicht zur Ausfällung oder Kristallisation des Antikörpers führen, sondern eine Ausfällung oder Kristallisation erst durch Zusatz von erfindungsgemäßen Präzipitationsreagenzien erfolgt.

Als "nicht-wässriges Medium" im Rahmen der Erfindung werden Öle oder Gemische von Ölen mit Wasser oder anderen geeigneten inerten Lösungsmitteln und anderen in Beispiel 1 genannten Agenzien verstanden, wie beispielsweise Stabilisatoren oder Hilfsstoffe, mit der Eigenschaft, dass erfindungsgemäße nicht-wässrige Medien allein nicht zur Ausfällung oder Kristallisation des Antikörpers führen, sondern eine Ausfällung oder Kristallisation erst durch Zusatz von erfindungsgemäßen Präzipitationsreagenzien erfolgt.

Bezüglich der erfindungsgemäßen anti-EGFR-Antikörper und im Rahmen der vorliegenden Erfindung, versteht man unter "biologisch aktiv", "nativ" und "wirksam", dass erfindungsgemäße anti-EGFR-Antikörper auch nach Überführung in erfindungsgemäße feste Formen und anschließender Wiederauflösung oder Resuspension ihre biologische Wirkung ausüben können, insbesondere die Bindung an EGFR, Inhibition der Bindung von Liganden, insbesondere EGF, an den EGFR, Modulation, insbesondere Inhibition der EGFR-vermittelten Signaltransduktion und Prophylaxe oder Therapie von EGFR-vermittelten Erkrankungen.

Insbesondere eine unproblematische Herstellung von Kristallen von erfindungsgemäßen anti-EGFR-Antikörpern war deshalb nicht zu erwarten, da Antikörper im Allgemeinen eine starke Tendenz zur Aggregation aufweisen, was den geordneten Einbau in ein Kristallgitter erschwert oder gar verhindert (McPherson A. (1999) Crystallization of Biological Macromolecules, 1 edn. Cold Spring Harbor Laboratory Press, New York). Trotz der für den Fachmann zu erwartenden Schwierigkeiten bei der Kristallisation erfindungsgemäßer Antikörper aufgrund der obengenannten Inhomogenität bezüglich Proteinstruktur, Glykosylierung und struktureller segmentaler Flexibilität auch innerhalb einer Antikörper-Spezies, werden überraschenderweise mit erfindungsgemäßen Verfahren ausgezeichnete Ergebnisse erzielt.

Die hohe Stabilität der erhaltenen Kristalle erfindungsgemäßer Antikörper ist auch dadurch gekennzeichnet, dass es weder zur Veränderung der Partikelform und des Partikelgrößenspektrums, was als kritisch im Hinblick auf immunogene Nebenwirkungen anzusehen wäre, noch zu Veränderungen in der Primärstruktur oder Sekundärstruktur des Proteins kommt. Das erfindungsgemäße Verfahren bietet somit den Vorteil, dass vorzugsweise die Partikelgröße der erhaltenen Kristalle kontrollierbar ist und vorzugsweise stabile dreidimensionale Kristalle erhalten werden- Die Kristallgröße kann im Bereich von µm und nm liegen.

anti-EGFR-Antikörper: Erfindungsgemäße anti-EGFR-Antikörper sind vorzugsweise monoklonal und muriner oder humaner Herkunft, besonders bevorzugt sind sie muriner Herkunft und chimär oder humanisiert. Bei dem gegen den Rezeptor des epidermalen Wachstumsfaktors (EGFR) gerichteten Antikörper handelt es sich besonders bevorzugt um Mab C225 (Cetuximab) oder Mab h425 (EMD 72000) und/oder deren Varianten oder Fragmente. Weitere gegen den EGFR gerichtete Antikörper sind z.B. in der EP0586002 sowie in J. Natl. Cancer Inst. 1993, 85: 27-33 (Mab 528) beschrieben.

Mab C225 (Cetuximab, Erbitux^{™}): Mab C225 (Cetuximab) ist ein klinisch erprobter Antikörper, der an den EGF-Rezeptor bindet. Mab C225 (Cetuximab) ist ein chimärer Antikörper, dessen variable Regionen murinen und dessen konstante Regionen humanen Ursprungs sind. Er wurde erstmals von Naramura et al., Cancer Immunol. Immunotherapy 1993, 37: 343-349 sowie in WO 96/40210 A1 beschrieben.

Mab h425 (EMD 72000): Mab h425 (EMD 72000) ist ein humanisierter monoklonaler Antikörper (Mab), der aus dem murinen anti-EGFR-Antikörper 425 (Mab 425) erhalten wurde (EP0531472). Der murine monoklonale Antikörper Mab 425 wurde in der humanen Karzinomzelllinie A431 entwickelt, da er hier an ein extrazelluläres Epitop des epidermalen Wachstumsfaktorrezeptor (EGFR) bindet. Es wurde gefunden, dass er die Bindung von EGF inhibiert (Murthy et al., 1987). Erhöhte Expression von EGFR wird in malignen Geweben aus unterschiedlichen Quellen gefunden, so dass Mab 425 ein möglicher Wirkstoff zur Diagnose und therapeutischen Behandlung von humanen Tumoren ist. So wurde gefunden, dass Mab 425 in vitro Tumorzytotoxizität vermittelt und in vitro das Tumorwachstum von Zellinien epidermoider und colorektaler Karzinome unterdrückt (Rodeck et al., 1987). Außerdem konnte gezeigt werden, dass Mab 425 an Xenografts von humanen malignen Gliomen in Mäusen bindet (Takahashi et al., 1987). Seine humanisierten und chimären Formen sind z.B. aus EP0531472; Kettleborough et al., Protein Engineering 1991, 4: 773-783; Bier et al., Cancer Chemother Pharmacol. 2001, 47: 519-524; Bier et al., Cancer Immunol. Immunother. 1998, 46: 167-173 bekannt. Mab h425 (EMD 72000) ist dabei ein in der klinischen Phase I/II befindlicher humanisierter Antikörper (h425), dessen konstanter Bereich sich aus einer κ und einer humanen γ-1-Kette zusammensetzt (EP0531472).

Humane anti-EGFR-Antikörper können nach der XenoMouse-Technologie bereitgestellt werden, wie in der WO9110741, W09402602, WO9633735 beschrieben. Ein gemäß dieser Technologie hergestellter, in der klinischen Erprobung befindlicher Antikörper ist beispielsweise auch ABX-EGF (Abgenix, Crit. Rev. Oncol. Hematol. 2001, 38: 17-23; Cancer Research 1999, 59: 1236-43).

Antikörper Antikörper oder Immunglobulin wird im Rahmen der vorliegenden Erfindung im breitesten Umfang verwendet und betrifft insbesondere polyklonale Antikörper und multispezifische Antikörper (z.B. bispezifische Antikörper) und besonders bevorzugt intakte monoklonale Antikörper (Mab), die biologisch wirksam sind, sowie deren Varianten und Fragmente. Es sind auch Heteroantikörper, die aus zwei oder mehreren Antikörpern oder deren Fragmenten bestehen und/oder verschiedene Bindungsspezifitäten aufweisen und miteinander verbunden sind, umfasst.

In Abhängigkeit von der Aminosäuresequenz ihrer konstanten Regionen können Antikörper verschiedenen "Antikörper- (Immunglobulin-) Klassen zugeordnet werden: IgA, IgD, IgE, IgG und IgM. Mehrere davon können weiter in Subklassen (Isotypen) unterteilt werden, beispielsweise IgG1, IgG2, IgG3, IgG4, IgA1 und IgA2. Antikörper haben gewöhnlich ein Molekulargewicht von etwa 150 kDa, bestehen aus zwei identischen leichten Ketten (L) und zwei identischen schweren Ketten (H). Monokonale Antikörper werden aus einer Population homogener Zellen erhalten. Sie sind hochspezifisch und gegen ein einziges Epitop gerichtet, während polyklonale Antikörper verschiedene Antikörper umfassen, die gegen unterschiedliche Epitope gerichtet sind. Verfahren zur Herstellung monoklonaler Antikörper umfassen beispielsweise die von Kohler und Milstein (Nature 256, 495 (1975)) und in Burdon et al., (1985) "Monoclonal Antibody Technology, The Production and Characterization of Rodent and Human Hybridomas", Eds, Laboratory Techniques in Biochemistry and Molecular Biology, Volume 13, Elsevier Science Publishers, Amsterdam beschriebene Hybridommethode. Sie können insbesondere durch bekannte rekombinante DNA-Techniken hergestellt werden (siehe z.B. US4816567). Monoklonale Antikörper können auch aus Phagenantikörperbibliotheken isoliert werden beispielsweise mithilfe der in Clackson et al. (Nature, 352:624-628 (1991)) und Marks et al. (J. Mol. Biol., 222:58, 1-597(1991)) beschriebenen Techniken.

Varianten und Fragmente: Bei Varianten (Muteine) von Antikörpern handelt es sich um strukturverwandte Proteine, beispielsweise solche, die durch Modifikation der Primärsequenz (Aminosäuresequenz), durch Glycoengineering (Varianten der Glykosylierungsstellen bzw-strukturen, auch deglykosylierte Proteine), durch PEGylierung, durch Herstellung in modifizierten Wirtszellen oder durch andere Techniken erhalten werden können. Erfindungsgemäße Varianten sind hierbei nicht auf die vorstehenden Beispiele beschränkt, sondern umfassen alle dem Fachmann bekannten Varianten erfindungsgemäßer Antikörper.

Bei Fragmenten (Teilsegmenten) von Antikörpern handelt es sich um Spaltungsprodukte von Antikörpern, die beispielsweise durch limitierte enzymatische Verdauung mithilfe von Papain, Pepsin und Plasmin oder durch gentechnische Herstellung der Teilsegmente gewonnen werden. Typische Teilsegmente sind beispielsweise das bivalente F(ab')₂-Fragment, das monovalente Fab-Fragment sowie das Fc-Fragment. (Lottspeich F. , H. Zorbas (ed.). Bioanalytik, Heidelberg; Berlin:Spektrum Akademischer Verlag GmbH, (1998) pp.1035). Erfindungsgemäße Fragmente sind hierbei nicht auf die vorstehenden Beispiele beschränkt, sondern umfassen alle dem Fachmann bekannten Fragmente erfindungsgemäßer Antikörper.

Pharmazeutische Zubereitung: Die Begriffe pharmazeutische Formulierung und pharmazeutische Zubereitung werden im Rahmen der vorliegenden Erfindung als Synonyme verwendet.

Wie hier verwendet bezieht sich "pharmazeutisch verträglich" auf Arzneimittel, Präzipitationsreagezien, Trägerstoffe, Hilfsstoffe, Stabilisatoren, Lösungsmittel und sonstige Agenzien, die die Verabreichung der daraus erhaltenen pharmazeutischen Zubereitungen ohne unerwünschte physiologische Nebenwirkungen, wie Übelkeit, Schwindel, Verdauungsprobleme o.ä. an ein Säugetier ermöglichen .

Bei pharmazeutischen Zubereitungen zur parenteralen Verabreichung besteht die Forderung nach Isotonie, Euhydrie sowie nach Verträglichkeit und Sicherheit der Formulierung (geringe Toxizität), der eingesetzten Hilfsstoffe und des Primärpackmittels. Überraschenderweise haben erfindungsgemäße feste Formen von anti-EGFR-Antikörpern vorzugsweise den Vorteil, dass eine direkte Verwendung möglich ist, da als Präzipitationsreagenzien physiologisch unbedenkliche Agenzien verwendet werden und vor Verwendung der erfindungsgemäßen festen Formen in pharmazeutischen Formulierungen somit keine weiteren Reinigungsschritte zum Entfernen toxikologisch bedenklicher Agenzien, wie beispielsweise hohe Konzentrationen organischer Lösungsmittel oder anderer toxikologisch bedenklicher Hilfsstoffe, erforderlich sind. Somit ist die Herstellung erfindungsgemäßer fester Formen von anti-EGFR-Antikörpern bei vorzugsweise gleichzeitig hoher Ausbeute an nativem und pharmazeutisch unbedenklichem Protein hoher Reinheit, vorzugsweise einfach, zeitsparend und kostengünstig.

Gegenstand der Erfindung ist deshalb auch ein Verfahren zur Herstellung einer festen Form eines erfindungsgemäßen anti-EGFR-Antikörpers und/oder einer seiner Varianten und/oder Fragmente, welche durch Auflösen oder Suspendieren in wässrigem Medium zu biologisch aktivem Antikörperprotein führt, dadurch gekennzeichnet, dass der in wässriger Lösung gelöste oder suspendierte Antikörper und/oder seine Varianten und/oder Fragmente durch ein Präzipitationsreagenz ausgefällt werden und das Ausfällungsprodukt abgetrennt wird. Als Präzipitationsreagenzien werden im erfindungsgemäßen Verfahren vorzugsweise Polymere, besonders bevorzugt Polyethylenglycol (PEG), Salze, besonders bevorzugt Ammoniumsulfat, oder organische Lösungsmittel, besonders bevorzugt Ethanol verwendet.

Eine erfindungsgemäße feste Form eines anti-EGFR-Antikörpers kann hergestellt werden, indem einer Lösung, die erfindungsgemäße Antikörper enthält, die in Beispiel 1 genannten erfindungsgemäßen Präzipitationsreagenzien, vorzugsweise Polymere, wie besonders bevorzugt Polyethylenglykol (PEG) in einer Konzentration von 0,1 bis 99,9 % (w/v), mit einem durchschnittlichen Molekulargewicht von 200 - 80.000, bevorzugt 400 bis 20.000, besonders bevorzugt 400 - 8.000; Salze, wie besonders bevorzugt Ammoniumsulfat in einer Konzentration von 0,1 - 4,5 M, Natriumacetat-Trihydrat in einer Konzentration von 0,1 -4,5 M, triNatriumcitrat Dihydrat in einer Konzentration von 0,1-1,5 M, Kaliumphosphat in einer Konzentration von 0,1 - 1,2 M, Kaliumchlorid in einer Konzentration von 0,1 bis 4,7 M, Natriumchlorid in einer Konzentration von 0,1 bis 6,1 M, di-Kaliumhydrogenphosphat in einer Konzentration von 0,1 bis 3,0 M, di-Natriumhydrogenphosphat Dihydrat in einer Konzentration von 0,1 bis 0,5 M oder organische Lösungsmittel, wie besonders bevorzugt Ethanol in einer Konzentration von 0,1 - 99,9% (v/v), oder deren Mischungen, sowie Hilfsstoffe, Puffer und/oder Stabilisatoren in einer Batch-Methode zugefügt werden und das Gemisch bei in Beispiel 1 genannten pH-Werten und Temperaturen inkubiert wird. Zweckmäßigerweise wird hierzu einer Lösung mit einer definierten Konzentration an EGFR-Antikörper (0,01 bis 150 mg/ml, bevorzugt 2 bis 100 mg/ml, besonders bevorzugt ca. 5-20 mg/ml), wie sie bei dessen Herstellung gewonnen wird, mit definierten Volumina an Stammlösungen, die die in Beispiel 1 genannten Präzipitationsreagenzien, Hilfsstoffe, Puffer und/oder Stabilisatoren in definierter Konzentration enthalten, versetzt und gegebenenfalls mit Wasser oder Puffer (beispielsweise Citrat oder Phosphatpuffer, in einer Konzentration von 1mM bis 200 mM, bevorzugt 2 bis 20mM, besonders bevorzugt ca. 10 mM; auch unter Zusatz von Isotonisierungsmitteln wie beispielsweise Kaliumchlorid, Natriumchlorid in einer Konzentration von 1 -1000 mM, bevorzugt 40 mM - 310 mM) auf die vorberechnete Konzentration verdünnt. Alternativ können erfindungsgemäße Präzipitationsreagenzien, Hilfsstoffe, Puffer und Stabilisatoren auch in fester Form zugesetzt werden. Liegt der Antikörper selbst in festem Aggregatszustand vor, beispielsweise als Lyophilisat, können die erfindungsgemäßen festen Formen von anti-EGFR-Antikörpern hergestellt werden, indem erfindungsgemäße Antikörper zunächst in Wasser oder in einer einen oder mehrere weitere Inhaltsstoffe enthaltenden wässrigen Lösung gelöst werden und anschließend mit definierten Volumina an Stammlösungen, die die in Beispiel 1 genannten Präzipitationsreagenzien, Hilfsstoffe, Puffer und/oder Stabilisatoren in definierter Konzentration enthalten, versetzt werden. Erfindungsgemäße Präzipitationsreagenzien, Hilfsstoffe, Puffer und/oder Stabilisatoren können zudem auch in festem Aggregatszustand zugesetzt werden. Zweckmäßigerweise können erfindungsgemäße Antikörper auch direkt in einer alle Präzipitations-reagenzien, Hilfsstoffe, Puffer und/oder Stabilisatoren enthaltenden Lösung gelöst werden.

Die Erfindung umfasst auch alle dem Fachmann bekannten und denkbaren Hydrate, Salze und Derivate der vorstehend genannten Agenzien.

Vorteilhaft kann eines oder mehrere der in der Erfindung genannten Agenzien während oder nach Beendigung des Präzipitationsverfahrens zugegeben und gegebenenfalls wieder entfernt werden, um beispielsweise einen zusätzlichen Reinigungsschritt auszuführen.

Vorteilhaft kann eines oder mehrere der in der Erfindung genannten Präzipitationsreagenzien, Hilfsstoffe, Puffer und/oder Stabilisatoren während oder nach Beendigung des Herstellungsverfahrens des Antikörpers zugegeben werden. Bevorzugt kann dies dadurch erfolgen, indem der erfindungsgemäße Antikörper im letzten Schritt der nach seiner Herstellung erfolgenden Aufreinigung direkt in einer wässrigen Lösung gelöst wird, die einen, mehrere oder alle weiteren Präzipitationsreagenzien, Hilfsstoffe, Puffer und/oder Stabilisatoren enthält. Dann müssen zur Herstellung einer erfindungsgemäßen pharmazeutischen Zubereitung die jeweiligen weiteren lnhaltsstoff/e nur noch in jeweils geringerer Menge oder gar nicht zugesetzt werden. Besonders bevorzugt ist, wenn der jeweilige Inhaltsstoff im letzten Schritt der Aufreinigung des Antikörpers, die seiner Herstellung folgt, in einer alle weiteren Präzipitationsreagenzien, Hilfsstoffe, Puffer und/oder Stabilisatoren enthaltenden wässrigen Lösung gelöst wird. So kann vorteilhafterweise eine direkt abzufüllende oder zu lyophilisierende Lösung erhalten werden. Die so erhaltene, den jeweiligen Antikörper enthaltende Lösung wird auf einen pH-Wert von 4 bis 10, bevorzugt pH 5 bis 9 eingestellt, sterilfiltriert und je nach Bedarf gefriergetrocknet.

Die Umsetzung erfolgt nach dem Fachmann bekannten Methoden in einem geeigneten Lösungsmittel, insbesondere in einem inerten Lösungsmittel. Als inerte Lösungsmittel eignen sich Ethanol, Glycerol, Gemische mit Wasser oder reines oder andere Hilfsstoffe, wie beispielsweise Salze mit puffernder oder isotonsierender Wirkung. enthaltendes Wasser. Besonders bevorzugt ist Wasser.

Besonders bevorzugt kann das hier beschriebene Verfahren im "batch"-Format durchgeführt werden. Nach erfindungsgemäßem Verfahren hergestellte erfindungsgemäße feste Formen von anti-EGFR-Antikörpem und/oder seiner Varianten und/oder Fragmente können besonders bevorzugt durch Auflösen oder Suspendieren in wässrigem Medium in biologisch aktives Antikörperprotein überführt werden. Dazu werden besonders bevorzugt ein in wässriger Lösung gelöster oder suspendierter Antikörper und/oder eine seiner Varianten und/oder Fragmente durch ein in Beispiel 1 genanntes Präzipitationsreagenz ausgefällt und das Ausfällungsprodukt abgetrennt.

Je nach Wahl der Konzentration des Präzipitationsreagenzes werden entweder amorphe Präzipitate oder Kristalle erhalten. Vorzugsweise werden bei höherer Konzentration des Präzipitationsreagenzes Präzipitate, bei niedrigerer Konzentration des Präzipitationsreagenzes dagegen Kristalle erhalten. Durch geeignete Wahl, der Konzentration des Prazipitationsreagenzes, der Konzentration des Proteins, der übrigen erfindungsgemäßen Agenzien, des pH-Werts und der Temperatur lässt sich so die Umsetzung in die gewünschte Richtung lenken. In den Beispielen 2 und 3 sind beispielhafte Kristallisationsbedingungen für Mab C225 (Erbitux^{™}) aufgeführt, in den Beispielen 4 und 5 beispielhafte Präzipitationsbedingungen. Das erfindungsgemäße Präzipitations-verfahren und das erfindungsgemäße Kristallisationsverfahren können auch kombiniert werden.

Geeignete Reaktionstemperaturen liegen bei Temperaturen von -10 bis 40°C, vorzugsweise bei 0 bis 25°C und ganz besonders bevorzugt bei 4 bis 20°C. Bevorzugt wird bei einem Druck von 1 bis 20 bar gearbeitet, besonders bevorzugt bei Normaldruck. Bevorzugt wird bei einem pH-Wert von 4 bis 10 gearbeitet. Die Dauer der Umsetzung hängt von den gewählten Reaktionsbedingungen ab. In der Regel beträgt die Reaktionsdauer 0.5 Stunden bis 10 Tage, vorzugsweise 1 bis 24 Stunden, besonders bevorzugt 2 bis 12 Stunden.

Unter Solvaten der erfindungsgemäßen festen Formen werden Anlagerungen von inerten Lösungsmittelmolekülen an die erfindungsgemäßen festen Formen verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Ethanol.

Die erhaltenen erfindungsgemäßen festen Formen von anti-EGFR-Antikörpern können von der entsprechenden Lösung, in der sie hergestellt werden, getrennt werden (z.B. durch Zentrifugation und Waschen) und können nach der Trennung in einer anderen Zusammensetzung gelagert werden, oder sie können direkt in der Herstellungslösung verbleiben. Die erhaltenen erfindungsgemäßen festen Formen können auch zur jeweiligen Verwendung in gewünschte Lösungsmittel aufgenommen werden. Die erfindungsgemäßen festen Formen von anti-EGFR-Antikörper sind vorzugsweise nach Wiederauflösung oder Resuspension biologisch aktiv und es kommt bei erfindungsgemäßen Verfahren vorzugsweise nicht zu einer Denaturierung der Antikörper. So bleibt die biologische Wirksamkeit des Proteins vorzugsweise erhalten.

Ebenso wurde überraschend gefunden, dass sich mithilfe erfindungsgemäßer fester Formen von anti-EGFR-Antikörpern vorzugsweise stabile pharmazeutische Formulierungen herstellen lassen. Diese Formulierungen weisen vorzugsweise eine höhere Stabilität gegenüber physikochemischen Einflüssen wie beispielsweise Oxidation, mechanischen Belastungen, ungünstigen pH-Werten und Temperaturen auf als herkömmliche Proteinlösungen von Antikörpern. Üblicherweise ist eine vergleichbare Stabilität sonst nur durch kosten- und zeitaufwendige Methoden zu erreichen, wie beispielsweise der Zusatz von Stabilisatoren, eine kühle Lagerung, Einfrieren oder Gefriertrocknung. Die hohe Stabilität ermöglicht vorzugsweise eine einfachere und kostengünstigere Lagerung, Transport und Herstellung pharmazeutisch wertvoller Formulierungen, wie beispielsweise ready-to-use-Formulierungen, Formulierungen mit verzögerter Wirkstofffreisetzung oder kontrollierter Freisetzung über einen längeren Zeitraum hinweg.

Ein weiterer Gegenstand der Erfindung sind deshalb erfindungsgemäße feste Formen von anti-EGFR-Antikörpem als lagerstabile Arzneimittel.

Ein besonders bevorzugter Gegenstand der Erfindung sind auch pharmazeutische Zubereitungen enthaltend wenigstens eine erfindungsgemäße feste Form eines anti-EGFR-Antikörpers in präzipitierter nicht-kristalliner, präzipitierter kristalliner oder in gelöster oder suspendierter Form, sowie gegebenenfalls Träger- und/oder Hilfsstoffe und/oder weitere pharmazeutische Wirkstoffe.

Somit können erfindungsgemäße pharmazeutische Zubereitungen erfindungsgemäße feste Formen in präzipitierter Form, d.h. als Präzipitat oder als Kristall, oder in wiederaufgelöster oder resuspendierter Form enthalten. Gegenstand der Erfindung sind deshalb auch pharmazeutische Zubereitungen, enthaltend wenigstens ein Präzipitat und/oder Kristall eines anti-EGFR-Antikörpers, bevorzugt eines monoklonalen anti-EGFR-Antikörpers, besonders bevorzugt von Mab C225 (Cetuximab) oder Mab h425 (EMD 72000) und/oder deren Varianten oder Fragmenten in präzipitierter, wiederaufgelöster oder suspendierter Form sowie gegebenenfalls Träger- und/oder Hilfsstoffe und/oder weitere pharmazeutische Wirkstoffe.

Vorzugsweise ermöglichen es die erfindungsgemäßen festen Formen von anti-EGFR-Antikörpern, hochkonzentrierte Formulierungen herzustellen, ohne dass es zu ungünstigen unerwünschten Aggregationen der erfindungsgemäßen Antikörper oder zu unerwünscht hoher Viskosität kommt, wie sie bei gewöhnlichen, hochkonzentrierten Proteinlösungen beobachtet werden kann. So lassen sich mithilfe erfindungsgemäßer fester Formen von anti-EGFR-Antikörpern mit wässrigen Lösungsmitteln oder in wässrigen Medien applikationsfertige Lösungen mit hohem Wirkstoffanteil wiederauflösen oder resuspendieren. In jüngerer Zeit werden vermehrt möglichst hochkonzentrierte Formulierungen von Proteinwirkstoffen gefordert. Die meisten Antikörper, die zur Therapie eingesetzt werden, werden im Bereich mg/kg dosiert. Eine hohe Dosis und geringe zu applizierende Volumina (z.B. ca. 1 bis 1,5 ml bei subkutaner Applikation) zeigen den Bedarf an hochkonzentrierten Proteinzubereitungen mit Konzentrationen von mehr als 100 mg/ml. Außerdem können hochkonzentrierte Proteinformulierungen in präklinischen Tests zur Untersuchung der Unbedenklichkeit und Wirksamkeit in-vitro und in-vivo (am Tiermodell), in klinischen Tests zur Untersuchung der Unbedenklichkeit und Wirksamkeit beim Menschen und in der klinischen Anwendung des Produktes (insbesondere bei der subkutanen Applikation) erhebliche Vorteile aufweisen. Ihre Vorteile bestehen insbesondere darin, dass ein geringeres Volumen der Zubereitung angewendet werden muss. Im Gegensatz zur Infusion oder Injektion von relativ niedrig konzentrierten Proteinarzneimitteln ist dadurch z.B. eine subkutane Applikation von Proteinarzneimitteln für den Patienten möglich. Die subkutane Applikation von Proteinarzneimitteln kann verschiedene Gründe haben. Beispielsweise kann ein spezielles Targeting erwünscht sein, welches in Zusammenhang mit einem therapeutischen Fenster" steht. Des weiteren hat eine subkutane Applikation den Vorteil, dass der Patient selbst die Applikation vornehmen kann, ohne auf ärztliches Personal angewiesen zu sein. Das Beispiel des Insulins zeigt deutlich diese Vorteile. Da die Injektionen zur subkutanen Applikation jedoch maximal 1 - 1,5 ml betragen können, sind häufig hochkonzentrierte Proteinformulierungen mit mehr als 100 mg/ml nötig.

Überraschenderweise lassen sich mithilfe erfindungsgemäßer fester Formen von anti-EGFR-Antikörpern hochkonzentierte pharmazeutische Zubereitungen erhalten, die in einer flüssigen Formulierung Proteinkonzentrationen von bevorzugt 10-200 mg/mi, besonders bevorzugt von 50 -150 mg/ml ermöglichen.
Dies war nicht zu erwarten, da bei hoch konzentrierten Proteinformulierungen die Tendenz zur Instabilität weitaus größer ist als bei verdünnten Proteinformulierungen (Fields, G., Alonso, D., Stiger, D., Dill, K. (1992) "Theory for the aggregation of proteins and copolymers." J. Phys. Chem. 96, 3974-3981). Bei einer hohen Proteinkonzentration ist die "Packungsdichte" der Proteinmoleküle erhöht. Eine vermehrte Anzahl an Kollisionen ist demnach anzunehmen und es kann zu gelegentlichen Protein-Assoziationen kommen. Dieser Prozess erfolgt in der Regel durch Nukleations- und Wachstumsmechanismen, bei der die kritischen Nuklei oft lösliche assoziierte Proteine darstellen, die sich jedoch schnell in unlösliche Proteinpräzipitate (denaturiertes Protein) umwandeln können (Reithel, J.F. (1962) "The dissociation and association of protein structures", Adv. Protein Chem. 18, 123). Die Grösse der Proteinaggregate erhöht sich mit steigender Proteinkonzentration, wie für das β-Lactoglobulin bereits gezeigt werden konnte (Roefs, S.P.F.M., De Kruif, K.G. (1994) "A model for the denaturation and aggregation of β+-lactoglobulin." Eur. J. Biochem. 226, 883-889)

Die Grenze bei bekannten hochkonzentrierten Formulierungen von Immunglobulinen liegt bei gebrauchsfertigen flüssigen Formulierungen von Antikörpern normalerweise bei 2 - 50mg/mL (Humira^{®}).
Mit den erfindungsgemäßen festen Formen lassen sich jedoch stabile hochkonzentrierte Formulierungen herstellen, was nicht zu erwarten war. Somit kann durch den Schritt der Präzipitation oder Kristallisation eine hochkonzentrierte stabile Antikörperformulierung erhalten werden, die nach Resuspendierung oder Wiederauflösen eine veringerte Viskosität verglichen mit flüssigen Antikörperformulierungen gleicher Konzentration aufweist und dadurch die Handhabbarkeit bei der parenteralen Verabreichung erleichtert.

Gegenstand der Erfindung sind deshalb auch pharmazeutische Zubereitungen, enthaltend wenigstens eine erfindungsgemäße feste Form eines anti-EGFR-Antikörpers und/oder eine seiner Varianten und/oder Fragmente in präzipitierter nicht-kristalliner, präzipitierter kristalliner oder in gelöster oder suspendierter Form enthält, wobei der enthaltene Antikörper biologisch aktiv ist, dadurch gekennzeichnet, dass die Antikörperkonzentration bevorzugt 10 - 200 mg/ml, besonders bevorzugt 50 - 150 mg/ml beträgt.

Gegenstand der Erfindung ist auch ein Verfahren zur Herstellung einer erfindungsgemäßen hochkonzentrierten pharmazeutischen Zubereitung, dadurch gekennzeichnet, dass man wenigstens eine erfindungsgemäße feste Form eines anti-EGFR-Antikörpers und/oder eine seiner Varianten und/oder Fragmente in einer erfindungsgemäßen Lösung löst oder resuspendiert und die Antikörperkonzentration bevorzugt 10 - 200 mg/ml, besonders bevorzugt 50 -150 mg/ml beträgt.
Wässrige Zubereitungen können hergestellt werden, indem man erfindungsgemäße feste Formen von anti-EGFR-Antikörpern in einer wässrigen Lösung löst oder suspendiert und gegebenenfalls Hilfsstoffe zufügt. Zweckmäßigerweise wird hierzu einer Lösung oder Suspension mit einer definierten Konzentration an erfindungsgemäßen festen Formen mit definierten Volumina an Stammlösungen, die die genannten weiteren Hilfsstoffe in definierter Konzentration enthalten, versetzt und ggf. mit Wasser auf die vorberechnete Konzentration verdünnt. Alternativ können die Hilfsstoffe in fester Form zugesetzt werden. Die erhaltene wässrige Lösung oder Suspension kann anschließend mit den jeweils erforderlichen Mengen an Stammlösungen und/oder Wasser versetzt werden. Zweckmäßigerweise können erfindungsgemäße feste Formen von anti-EGFR-Antikörpern auch direkt in einer alle weiteren Hilfsstoffe enthaltenden Lösung gelöst oder suspendiert werden.

Vorteilhaft können aus den erfindungsgemäßen festen Formen durch die Rekonstitution mit wässrigen Lösungsmitteln antikörperhaltige Lösungen oder Suspensionen mit einem pH-Wert von 4 bis 10, bevorzugt mit einem pH-Wert von 5 bis 9, und einer Osmolalität von 250 bis 350 mOsmol/kg hergestellt werden.. Die resuspendierte oder wieder gelöste Zubereitung kann somit weitgehend schmerzfrei intravenös, intraarteriell und auch subkutan direkt verabreicht werden. Daneben kann die Zubereitung auch Infusionslösungen, wie z.B. Glukoselösung, isotonische Kochsalzlösung oder Ringerlösung, die auch weitere Wirkstoffe enthalten können, zugesetzt werden, so dass auch größere Wirkstoffmengen appliziert werden können.

Erfindungsgemäße pharmazeutische Zubereitungen können auch Mischungen erfindungsgemäßer Präzipitate und/oder Kristalle in präzipitierter bzw. kristalliner und/oder wiederaufgelöster oder resupsendierter Form enthalten.

Die erfindungsgemäßen Zubereitungen sind physiologisch gut verträglich, leicht herstellbar, exakt dosierbar und vorzugsweise über die Dauer der Lagerung sowie des Transports und bei mehrfachen Einfrier- und Auftauvorgängen hinsichtlich Gehalt, Zersetzungsprodukten und Aggregaten stabil. Sie können bei Kühlschranktemperatur (2-8°C) und bei Raumtemperatur (23-27°C) und 60 % relativer Luftfeuchtigkeit (r.F.) vorzugsweise über einen Zeitraum von mindestens drei Monaten bis zu zwei Jahren stabil gelagert werden. Überraschenderweise sind die erfindungsgemäßen Zubereitungen vorzugsweise auch bei höheren Temperaturen und Luftfeuchtigkeiten, beispielsweise bei einer Temperatur von 40°C und 75% relativer Luftfeuchtigkeit über mindestens sechs Monatestabil lagerbar.

Beispielsweise können erfindungsgemäße feste Formen durch Trocknung stabil gelagert und bei Bedarf durch Lösung oder Suspension in eine gebrauchsfertige pharmazeutische Zubereitung überführt werden. Mögliche Methoden zur Trocknung sind zum Beispiel, ohne auf diese Beispiele beschränkt zu sein, Stickstoffgastrocknung, Vakuumofen-Trocknung, Lyophilization, Waschen mit organischem Lösungsmittel und anschließende Lufttrocknung, Flüssigbett-Trocknung, Wirbelschichttrocknung, Sprühtrocknung, Walzentrocknung, Lagentrocknung; Lufttrocknung bei Raumtemperatur und weitere Methoden.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat: verbesserte Heilbehandlung, Heilung, Prävention oder Beseitigung einer Krankheit, eines Krankheitsbildes, eines Krankheifszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Arzneimittel können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 800 mg, eines erfindungsgemäßen Wirkstoffs enthalten, je nach dem behandelten Krankheitszustand, dem Verabreichungsweg und dem Alter, Gewicht und Zustand des Patienten. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche Arzneimittel mit einem der im pharmazeutischen Fachgebiet allgemein bekannten Verfahren herstellen.

Arzneimittel lassen sich zur Verabreichung über einen beliebigen geeigneten Weg, beispielsweise auf oralem (einschließlich buccalem bzw. sublingualem), rektalem, pumonalen, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

Zur Verabreichung der erfindungsgemäßer Arzneimittel eignen sich vorzugsweise die parenterale Applikation. Im Falle der parenteralen Applikation sind die intravenöse und subkutane Applikation besonders bevorzugt. Im Falle der intravenösen Applikation kann die Injektion direkt oder auch als Zusatz zu Infusionslösungen erfolgen.

Insbesondere eignen sich Arzneimittel zur subkutanen Applikation, da sich mithilfe erfindungsgemäßer fester Formen von anti-EGFR-Antikörpern stabile, hochkonzentrierte Forumlierungen herstellen lassen. Dadurch lassen sich die für die parentrale oder subkutane Verabreichung erforderlichen hochkonzentrierten Formulierungen und geringe zu applizierende Volumina realisieren.

Die subkutane Applikation hat den Vorteil, dass der Patient ohne medizinische fachmännische Hilfe sich selbst das Arzneimittel verabreichen kann. Erfindungsgemäße feste Formen von anti-EGFR-Antikörpern eignen sich auch zur Herstellung von parenteral zu applizierenden Arzneimitteln mit kontrollierter, gleichmäßiger und/oder verzögerter Wirkstofffreisetzung (slow-release, sustained-release, controlled release). Die erfindungsgemäßen Präzipitate und/oder Kristalle liegen dabei vorzugweise in Suspension vor und lösen sich über einen längeren und/oder kontrollierten Zeitraum hinweg auf und liegen dann in nativem und wirksamen Zustand vor. Somit eignen sich erfindungsgemäße feste Formen von anti-EGFR-Antikörpem auch zur Herstellung von Depot-Formulierungen, die für den Patienten vorteilhaft sind, da eine Applikation nur in größeren Zeitintervallen notwendig ist. Erfindungsgemäße pharmazeutische Zubereitungen können auch direkt in den Tumor injiziert werden und so direkt am bestimmungsgemäßen Wirkort ihre Wirkung entfalten.

Zu den an die parenterale Verabreichung angepassten Arzneimitteln gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Die erfindungsgemäßen festen Formen von anti-EGFR-Antikörpern, gegebenenfalls in wiederaufgelöster Form lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen festen Formen von anti-EGFR-Antikörpern und deren Varianten in präzipitierter, wiederaufgelöster oder suspendierter Form können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die festen Formen von anti-EGFR-Antikörpern an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate, Polymilchcoglykolsäure, Polymere wie Konjugate zwischen Dextran und Methacrylate, Polyphosphoester, verschiedene Polysaccharide und Polyamine sowie Poly-ε-caprolacton, Albumin, Chitosan, Collagen oder modifizierte Gelatine und quervernetzte oder amphipatische Blockcopolymere von Hydrogelen, gekoppelt sein.

An die transdermale Verabreichung angepasste Arzneimittel können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste Arzneimittel können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten Arzneimittel gehören Augentropfen, wobei der Wirkstoff in einem geeigneten Träger, insbesondere einem wässrigen Lösungsmittel, gelöst oder suspendiert ist.

An die rektale Verabreichung angepasste Arzneimittel können in Form von Zäpfchen oder Einläufen dargereicht werden..

An die Verabreichung durch Inhalation angepasste Arzneimittel umfassen feinpartikuläre Stäube oder Nebel, die mittels verschiedener Arten von unter Druck stehenden Dosierspendem mit Aerosolen, Vemeblem oder Insufflatoren erzeugt werden können. Besonders bevorzugt im Rahmen der vorliegenden Erfindung sind Pulver erfindungsgemäßer fester Formen von anti-EGFR-Antikörpern zur Applikation als Inhalat.

An die vaginale Verabreichung angepasste Arzneimittel können als Pessare, Tampons, Cremes, Gele, Pasten, Schäume oder Sprayformulierungen dargereicht werden.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können.

Ein weiterer Gegenstand der Erfindung sind Sets (Kits) bestehend aus getrennten Packungen von
a) einer wirksamen Menge eines Präzipitats und/oder eines Kristalls eines anti-EGFR-Antikörpers, bevorzugt eines monoklonalen anti-EGFR-Antikörpers, besonders bevorzugt von Mab C225 (Cetuximab) oder Mab h425 (EMD 72000) und/oder deren Varianten oder Fragmenten in präzipitierter, wiederaufgelöster oder suspendierter Form, und
b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge einer erfindungsgemäßen festen Form, gegebenenfalls in wiederaufgelöster Form und einer wirksamen Menge eines weiteren Arzneimittelwirkstoffs gelöst oder in lyophylisierter Form vorliegt.

Eine therapeutisch wirksame Menge einer erfindungsgemäßen festen Form eines anti-EFGR-Antikörpers hängt von einer Reihe von Faktoren ab, einschließlich z.B. dem Alter und Gewicht, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge eines erfindungsgemäßen anti-EFGR-Antikörpers für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Die Bestimmung des geeigneten Antikörper-Titers erfolgt nach dem Fachmann bekannten Methoden. Die für die Verabreichung vorgesehe Dosierung ist im Allgemeinen ausreichend, um die gewünschte tumor-hemmende Wirkung zu erzielen. Die Dosierung sollte jedoch auch möglichst gering gewählt werden, so dass keine Nebenwirkungen, wie unerwünschte Kreuzreaktionen, anaphylaktische Reaktionen o.ä. auftreten.

Ein weiterer Gegenstand der Erfindung ist die Verwendung erfindungsgemäßer fester Formen zur Herstellung eines Medikaments, welches den biologisch aktiven Antikörper und/oder eine seiner Varianten und/oder Fragmente in präzipitierter nicht-kristalliner, präzipitierter kristalliner oder in gelöster oder suspendierter Form enthält.

Erfindungsgemäße Medikamente können insbesondere zur Prophylaxe und/oder zur Behandlung von Krankheiten und Krankheitszuständen verwendet werden. Gegenstand der Erfindung ist deshalb die Verwendung von erfindungsgemäßen festen Formen von anti-EGFR-Antikörpern, bevorzugt von monoklonalen anti-EGFR-Antikörpern, besonders bevorzugt von Mab C225 (Cetuximab) oder Mab h425 (EMD 72000) und/oder deren Varianten oder Fragmenten in präzipitierter, wiederaufgelöster oder suspendierter Form zur Herstellung eines Medikaments zur Behandlung und/oder Prophylaxe von Krankheiten.

In verschiedenen in vitro und in vivo Studien konnte gezeigt werden, dass die Blockade des EGFR durch Antikörper auf unterschiedlichen Ebenen gegen Tumore, beispielsweise durch Hemmung der Krebszellproliferation, Verringerung der tumorvermittelten Angiogenese, Induktion der Krebszellapoptose und Verstärkung der toxischen Wirkungen der Strahlentherapie und der herkömmlichen Chemotherapie.

Arzneimittel enthaltend feste Formen der erfindungsgemäßen Antikörper in wiederaufgelöster oder suspendierter Form können EGFR wirksam regulieren, modulieren oder hemmen und können deshalb zur Vorbeugung und/oder Behandlung von Erkrankungen im Zusammenhang mit unregulierter oder gestörter EGFR-Aktivität eingesetzt werden. Insbesondere lassen sich die erfindungsgemäßen festen Formen von anti-EGFR-Antikörpern deshalb bei der Behandlung gewisser Krebsformen einsetzen, sowie bei durch pathologische Angiogenese bedingten Erkrankungen wie diabetische Retinopathie oder Entzündungen.

Ein weiterer Gegenstand der Erfindung ist deshalb die Verwendung von erfindungsgemäßen festen Formen in präzipitierter, wiederaufgelöster oder resuspendierter Form zur Herstellung eines Medikaments zur Behandlung und/oder Prophylaxe von Krankheiten, die durch EGFR und/oder durch EGFR-vermittelte Signaltransduktion verursacht, vermittelt und/oder propagiert werden.

Erfindungsgemäße Arzneimittel eignen sich besonders zur Behandlung und/oder Prophylaxe von Krebs, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom), pathologische Angiogenese und metastatische Zellmigration. Die Arzneimittel sind ferner nützlich bei der Behandlung der Komplementaktivierungs-abhängigen chronischen Entzündung (Niculescu et al. (2002) Immunol. Res., 24:191-199) und durch HIV-1 (Human Immunodeficiency Virus Typ 1) induzierte Immunschwäche (Popik et al. (1998) J Virol, 72: 6406-6413).

Außerdem eignen sich die vorliegenden Arzneimittel als pharmazeutische Wirkstoffe für Säugetiere, insbesondere für den Menschen, bei der Behandlung von EGFR-bedingten Krankheiten. Der Ausdruck "EGFRbedingte Krankheiten " bezieht sich auf pathologische Zustände, die von der EGFR-Aktivität abhängig sind. EGFR ist entweder direkt oder indirekt an den Signaltransduktionswegen verschiedener Zellaktivitäten, darunter Proliferation, Adhäsion und Migration sowie Differenzierung beteiligt. Zu den Krankheiten, die mit EGFR-Aktivität assoziiert sind, zählen die Proliferation von Tumorzellen, die pathologische Gefäßneubildung, die das Wachstum fester Tumore fördert, Gefäß neubildung im Auge (diabetische Retinopathie, altersbedingte Makula-Degeneration und dergleichen) sowie Entzündung (Schuppenflechte, rheumatoide Arthritis und dergleichen).

Gewöhnlich werden die hier besprochenen Erkrankungen in zwei Gruppen eingeteilt, in hyperproliferative und nicht-hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immurischwächekrankheiten als nicht-krebsartige Krankheiten angesehen, von denen Arthritis, Entzündung, immunologische Krankheiten, Autoimmunkrankheiten und Immunschwächekrankheiten gewöhnlich als nicht-hyperproliferative Erkrankungen angesehen werden.

In diesem Zusammenhang sind Himkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich zur Gruppe der hyperproliferative Erkrankungen gezählt werden. Insbesondere krebsartiges Zellwachstum und insbesondere durch EGFR direkt oder indirekt vermitteltes krebsartiges Zellwachstum ist eine Erkrankung, die ein Ziel der vorliegenden Erfindung darstellt.

Es kann gezeigt werden, dass die erfindungsgemäßen Arzneimittel in einem Xenotransplantat-Tumor-Modell eine in vivo antiproliferative Wirkung aufweisen. Die erfindungsgemäßen Arzneimittel werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Arzneimittel sind nützlich für prophylaktische oder therapeutische Zwecke. Wie hierin verwendet, wird der Begriff "behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Arzneimittel vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Arzneimittel zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Empfänglichkeit einer bestimmten Zelle gegenüber der Behandlung mit den erfindungsgemäßen Arzneimitteln kann durch in vitro-Tests bestimmt werden. Typischerweise wird eine Kultur der Zelle mit einem erfindungsgemäßen Arzneimittel bei verschiedenen Konzentrationen für eine Zeitdauer inkubiert, die ausreicht, um den Wirkstoffen zu ermöglichen, Zelltod zu induzieren oder Migration zu inhibieren, gewöhnlich zwischen ungefähr einer Stunde und einer Woche. Zu in vitro-Tests können kultivierte Zellen aus einer Biopsieprobe verwendet werden. Die nach der Behandlung zurückbleibenden lebensfähigen Zellen werden dann gezählt.

Die Dosis variiert abhängig von den verwendeten spezifischen Arzneimitteln, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der spezifischen Zellzahl und kann fortgesetzt werden, bis im Wesentlichen keine unerwünschten Zellen mehr im Körper nachgewiesen werden.

Zur Identifikation von EGFR-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen und Krankheitszustände. Die Erkrankungen und Krankheitszustände, die durch erfindungsgemäße Arzneimittel behandelt, verhindert oder gelindert werden können umfassen die nachfolgend aufgeführten Erkrankungen und Krankheitszustände, sind jedoch nicht darauf beschränkt. Die erfindungsgemäßen Arzneimittel sind nützlich bei der Behandlung und/oder Prophylaxe einer Reihe verschiedener Erkrankungen und Krankheitszustände, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die vorliegende Erfindung umfasst die Verwendung der erfindungsgemäßen Arzneimittel zur Behandlung oder Vorbeugung von Krebs. Ein besonders bevorzugter Gegenstand der Erfindung ist deshalb die Verwendung erfindungsgemäßer fester Formen von anti-EGFR-Antikörpem zur Herstellung eines Medikaments zur Behandlung und/oder Prophylaxe von Tumoren und/oder Tumormetastasen, wobei der Tumor besonders bevorzugt aus der Gruppe ausgewählt ist, bestehend aus Gehimtumor, Tumor des Urogenitaltrakts, Tumor des lymphatischen Systems, Magentumor, Kehlkopftumor, Monozytenleukämie, Lungenadenokarzinom, kleinzelliges Lungenkarzinom, Bauchspeicheldrüsenkrebs, Glioblastom und Brustkarzinom, ohne darauf beschränkt zu sein.

Ein weiterer Gegenstand der Erfindung ist die Verwendung von erfindungsgemäßen Arzneimittel zur Herstellung eines Medikaments zur Behandlung von Krankheiten, ausgewählt aus der Gruppe der krebsartigen Erkrankungen bestehend aus Plattenepithelkrebs, Blasenkrebs, Magenkrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

Die erfindungsgemäßen Arzneimittel können an Patienten zur Behandlung von Krebs verabreicht werden. Die vorliegenden Arzneimittel hemmen die Tumorangiogenese und beeinflussen so das Wachstum von Tumoren (J. Rak et al. Cancer Research, 55:4575-4580, 1995). Die angiogenesehemmenden Eigenschaften der erfindungsgemäßen Arzneimittel eignen sich auch zur Behandlung bestimmter Formen von Blindheit, die mit Retina-Gefäßneubildung in Zusammenhang stehen.

Gegenstand der Erfindung ist deshalb außerdem die Verwendung erfindungsgemäßer fester Formen von anti-EGFR-Antikörpem in präzipitierter, wiederaufgelöster oder suspendierter Form zur Herstellung eines Medikaments zur Behandlung und/oder Prophylaxe von Krankheiten, die durch Angiogenese verursacht, vermittelt und/oder propagiert werden.

Eine derartige Krankheit, an der Angiogenese beteiligt ist, ist eine Augenkrankheit, wie Retina-Vaskularisierung, diabetische Retinopathie, attersbedingte Makula-Degeneration und dergleichen.

Gegenstand der Erfindung ist deshalb auch die Verwendung von erfindungsgemäßen festen Formen von anti-EGFR-Antikörpern in präzipitierter, wiederaufgelöster oder suspendierter Form zur Herstellung eines Medikaments zur Behandlung und/oder Prophylaxe von Krankheiten, ausgewählt aus der Gruppe bestehend aus Retina-Vaskularisierung, diabetischer Retinopathie, altersbedingter Makula-Degeneration und/oder Entzündungskrankheiten.

Ein weiterer Gegenstand der Erfindung ist die Verwendung von erfindungsgemäßen Arzneimitteln zur Behandlung und/oder Prophylaxe von Krankheiten, ausgewählt aus der Gruppe bestehend aus Psoriasis, rheumatoide Arthritis, Kontaktdermatitis, Spät-Typ der Überempfindlichkeitsreaktion, Entzündungen, Endometriose, Vernarbung, gutartiger Prostatahyperplasie, immunologischer Krankheiten, Autoimmunkrankheiten und Immunschwächekrankheiten.

Gegenstand der Erfindung ist auch die Verwendung von erfindungsgemäßen Arzneimitteln zur Behandlung und/oder Prophylaxe von Knochen-Pathologien, ausgewählt aus der Gruppe bestehend aus Osteosarkom, Osteoarthritis und Rachitis.

Weiterhin können die erfindungsgemäße Arzneimittel verwendet werden, um bei gewissen existierenden Krebschemotherapien und -bestrahlungen additive oder synergistische Effekte bereitzustellen, und/oder können dazu verwendet werden, um die Wirksamkeit gewisser existierender Krebschemotherapien und -bestrahlungen wiederherzustellen.

Gegenstand der Erfindung ist deshalb auch die Verwendung von erfindungsgemäßen festen Formen von anti-EGFR-Antikörpern in präzipitierter, wiederaufgelöster oder suspendierter Form zur Herstellung eines Arzneimittels zur Behandlung und/oder Prophylaxe von Krankheiten, wobei eine therapeutisch wirksame Menge einer erfindungsgemäßen festen Form in präzipitierter, wiederaufgelöster oder suspendierter Form in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferase-inhibitoren, 7) HMG-CoA-Reduktase-Inhibitoren, 8) HIV-Protease-Inhibitoren, 9) Reverse-Transkriptase-Inhibitoren, 10) Wachstumsfaktorrezeptor-Inhibitoren sowie 11) Angiogenese-inhibitoren verabreicht wird.

Gegenstand der Erfindung ist deshalb auch die Verwendung von erfindungsgemäßen festen Formen von anti-EGFR-Antikörpern in präzipitierter, wiederaufgelöster oder suspendierter Form zur Herstellung eines Arzneimittels zur Behandlung und/oder Prophylaxe von Krankheiten, wobei eine therapeutisch wirksame Menge einer erfindungsgemäßen festen Form in präzipitierter, wiederaufgelöster oder suspendierter Form in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferaseinhibitoren, 7) HMG-CoA-Reduktase-Inhibitoren, 8) HIV-Protease-Inhibitoren, 9) Reverse-Transkriptase-Inhibitoren, 10) Wachstumsfaktorrezeptor-Inhibitoren sowie 11) Angiogenese-Inhibitoren verabreicht wird.

Die erfindungsgemäßen Arzneimittel können so auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinbfocker (wie sie weiter unten definiert werden), wie αvβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Inhibitoren und ATP-Protonenpumpeninhibitoren enthalten.
Die vorliegenden Arzneimittel eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferaseinhibitoren, HMG-CoA-Reduktase-Inhibitoren, HIV-Protease-Inhibitoren, Reverse-Transkriptase-Inhibitoren, Wachstumsfaktor-Inhibitoren sowie Angiogeneseinhibitoren. Die vorliegenden Verbindungen eignen sich insbesondere zur gemeinsamen Anwendung mit Radiotherapie.
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxy-benzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Inhibitoren, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliemde Mittel, Mikrotubulin-Inhibitoren und Topoisomerase-Inhibitoren.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-1 0-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Inhibitorenn zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Inhibitoren sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethyl-amino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7.10-dihydroxy-2-(2-hydroxyethylaminomethyl)-6H-pyrazolo[4.5.1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)hamstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Inhibitorenn" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134). Erfindungsgemäße Arzneimittel können auch in Kombination mit allen weiteren dem Fachmann bekannten therapeutischen Antikörpern oder im Zusammenhang mit obengenannter Krankheiten geeigneten pharmazeutischen Wirkstoffen verabreicht werden.

Weiterhin können erfindungsgemäße feste Formen erfindungsgemäßer Antikörper in präzipitierter, wiederaufgelöster oder suspendierter Form zur Isolierung und zur Untersuchung der Aktivität oder Expression von EGFR verwendet werden. Außerdem eignen sie sich insbesondere zur Verwendung in diagnostischen Verfahren zu Erkrankungen im Zusammenhang mit unregulierter oder gestörter EGFR-Aktivität. Ein weiterer Gegenstand der Erfindung ist deshalb die Verwendung von erfindungsgemäßen festen Formen von anti-EGFR-Antikörpern in präzipitierter, wiederaufgelöster oder suspendierter Form als Aktivatoren oder Inhibitoren von EGFR, besonders bevorzugt als Inhibitoren von EGFR.

Für diagnostische Zwecke können erfindungsgemäße Antikörper beispielsweise radioaktiv markiert werden. Ein bevorzugtes Markierungsverfahren ist die Iodogen-Methode (Fraker et al., 1978). Für diagnostische Zwecke wird der Antikörper besonders bevorzugt als F(ab')2 Fragment verwendet. Dadurch werden hervorragende Ergebnisse erzielt, so dass keine Hintergrund-Subtraktion notwendig ist. Solche Fragmente können nach bekannten Methoden hergestellt werden (e.g., Herlyn et al., 1983). Im Allgemeinen wird ein Pepsin-Verdau in bei saurem pH-Wert duchgeführt und die Fragmente durch Protein A-Sepharose^{™} Chromatographie von unverdautem IgG und Fragmenten schwerer Ketten getrennt.

Die erfindungsgemäßen Präzipitate und/oder Kristalle zeigen bevorzugt eine vorteilhafte biologische Aktivität, die in Enzym-Assays, wie in den Beispielen beschrieben, leicht nachweisbar ist. In derartigen auf Enzymen basierenden Assays zeigen und bewirken die erfindungsgemäßen Antikörper bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

### Nachweismethoden:

In Bezug auf Nachweismethoden umfasst die vorliegenden Erfindung alle dem Fachmann oder aus der Literatur bekannten Nachweismethoden.

Kristalline Strukturen lassen sich beispielsweise anhand von Beugungsspektren in der Röntgendiffraktionsmessung nachweisen. Insbesondere lassen sich kristalline Strukturen erfindungsgemäßer Antikörper durch mikroskopische Untersuchungen unter Verwendung von Polarisationsfiltern nachweisen. Weitere Nachweismethoden umfassen, ohne darauf beschränkt zu sein, elektronenmikroskopische Aufnahmen.

Nachweise von Proteingröße, struktureller Integrität, Reinheit oder Glykosylierungsmuster der erfindungsgemäßen festen Formen in präzipitierter, wiederaufgelöster oder resuspendierter Form umfassen, ohne darauf beschränkt zu sein, SE-HPLC, Peptid-Mapping (Verdau), N-terminale Sequenzierung, SDS-Page, Tris-Glycin-Gradient-Gel (nicht-reduzierend), FTIR-Methode (Fourier transform infrared spectra), CD (circular dichroism), RAMAN Spektroskopie, Kohlenhydratfärbung (PAS-Methode), Oligosaccharid-Profiling, Bestimmung der Monosaccharid-Zusammensetzung oder isoelektrische Fokussierung.

Die Stabilität erfindungsgemäßer fester Formen oder Formulierungen lässt sich beispielsweise, ohne darauf beschränkt zu sein, mithilfe von Stabilitätsprogrammen bestimmen, z.B. einer Lagerung bei 25°C und 60% relativer Luftfeuchte und bei 40°C und 70% relativer Luftfeuchte über einen längeren Zeitraum hinweg und Bestimmung der Stabilität oder strukturellen Intergrität des Proteins in regelmäßigen Intervallen beispielsweise durch obengenannte Nachweismethoden (SE-HPLC, FT-IR; SDS-PAGE (reduzierend oder nicht-reduzierend)) nachweisen.

Nachweismethoden zur biologischen Aktivität oder Wirksamkeit erfindungsgemäßer fester Formen in präzipitierter kristaliner, präzipitierter nicht-kristalliner, gelöster oder suspendierter Form umfassen beispielsweise, ohne darauf beschränkt zu sein, ELISA, biologische Zellassays, FTIR oder CD.

Nachweismethoden verminderter Aggregationstendenz erfindungsgemäßer fester Formen Formen in präzipitierter kristaliner, präzipitierter nicht-kristalliner, gelöster oder suspendierter Form und damit der Möglichkeit hochkonzentrierte Formulierungen herzustellen, der Kristall- oder Präzipitatgröße umfassen beispielsweise, ohne darauf beschränkt zu sein, visuelle Kontrolle, Sub-visible particles Analyse, Nephelometrie oder Turbidimetrie, Dynamic Light Scattering Characterization.

Vor- und nachstehend sind alle Temperaturen in °C angegeben. In den nachfolgenden Beispielen bedeutet "übliche Aufarbeitung": Man gibt, falls erforderlich, Wasser hinzu und stellt, falls erforderlich, je nach Konstitution des Endprodukts auf pH-Werte zwischen 2 und 10 ein.

### Beispiel 1: Erfindungsgemäß verwendbare Agenzien und Präzipitations- und/oder Kristallisationsbedingungen

In nachfolgender Aufstellung sind jeweils alle denkbaren, dem Fachmann bekannten Hydrate, verwandten Salze und Derivate der genannten Verbindungen umfasst.

### 1. Präzpitationreagenzien:

### 1.1 Salze:

z.B. Natriumacetat Trihydrat:, Kaliumchlorid:, Natriumchlorid:, tri-Natriumcitrat Dihydrat:, di-Kaliumhydrogenphosphat:, di-Natriumhydrogenphosphat Dihydrat:, Ammoniumsuffat:, Ammoniumacetat, Ammoniumbromid, Ammoniumchlorid, tri-Ammoniumcitrat, di-Ammoniumhydrogencitrat, Ammoniumdihydrogenphosphat, di-Ammoniumhydrogenphosphat, di-Ammoniumtartrat, Citronensäure Monohydrat, Imidazol, Kaliumacetat, Kaliumbromid, tri-Kaliumcitrat Monohydrat, Kaliumdihydrogenphosphat, Kaliumsulfat, Magnesiumacetat Tetrahydrat, Magnesiumbromid Hexahydrat, Magnesiumchlorid Hexahydrat, Magnesiumsulfat Heptahydrat, Natriumdihydrogenphosphat Monohydrat, Natriumsulfat Decahydrat, Prolin, Succinsäure, Zinkacetat Dihydrat, Zinksulfat Heptahydrat

### 1.2 Polymere:

z.B. Polyethylenglykol (PEG):
Cyclodextrine, Dextran, Natriumcarboxymethylcellulose (Na-CMC), Poloxamer, Polyacrylsäure, Polyethylenglykolmonomethylether (mPEG), Polypropylenglykol (PPG), Polyvinylalkohol (PVA), Polyvinylpyrrolidon (PVP)

### 1.3 Organische Lösungsmittel:

z.B. Ethanol, Glycerol

### 2. Hilfsstoffe:

z.B. Viskositätsändemde Stoffe, Detergenzien (z.B. Tween®, Solutol® HS 15, Pluronic®, Cremophor® EL, Avacel® 20, Polyoxyethylensorbitanmonooleat), Reduktionsmittel (Glutathion, 2-Mercaptoethanol, Dithiothreitol), Komplexbildner (EDTA, EGTA)

### 3. Puffer:

z.B. Phosphatpuffer: Na- (oder K-)Phosphat; auch mit Zusatz von Isotisierungsmittel (z.B. Na-(oder K-)CI ), möglicher pH ca 6,0 - 8,2 Citrat-Puffer: Na-Citrat oder Citronensäure, möglicher pH ca 2,2 - 6,5;. auch mit Zusatz von Isotonisierungsmitteln (z.B. NaCl); auch andere Salze sind zur Isotonisiefung denkbar.
Succinat-Puffer: pH ca 4.8 - 6.3
Acetat-Puffer: Natriumacetat, pH ca 2.5 - 6.
Histidin-Puffer: pH ca 6,0 - 7.8
Glutaminsäure: pH 8,0 bis 10,2 -
Glycin (N,N-bis(2-Hydroxyethyl)glycin): pH ca. 8,6 bis 10,6
Glycinat-Puffer: pH ca 6.5 - 7.5
Imidazol: pH 6,2 bis 7,8
Kaliumchlorid: pH ca. 1,0 bis 2,2
Lactat-Puffer: pH ca 3.0 - 6.0
Maleat-Puffer: pH ca 2.5 - 5.0
Tartrat-Puffer: pH ca 3.0 - 5.0
Tris: pH ca 6.8 - 7.7
Phosphat-Citrat-Puffer

### 4. pH Bereich:

Der theoretisch denkbare pH-Wert ist pH 4 -10, bevorzugt ist pH 5 - 9

### 5. Temperaturbereich:

Der theoretisch denkbare Temperaturbereich ist die Temperatur von -10°C bis 40°C; bevorzugt 0 - 25°C, besonders bevorzugt 4°C und 20°C.

### 6. Stabilisatoren:

### 6.1 Aminosäuren:

z.B. Arginin, Ornithin, Lysin, Histidin, Glutaminsäure, Asparaginsäure, Isoleucin, Leucin, Alanin, Phenylalanin, Tyrosin, Tryptophan, Methionin, Serin, Prolin

### 6.2 Zucker und Zuckeralkohole:

z.B. Saccharose, Lactose, Glucose, Mannose, Maltose, Galactose, Fructose, Sorbose, Raffinose, Trehalose, Glucosamin, N-Methylglucosamin, Galactosamin, Neuraminsäure

### 6.3 Antioxidantien:

z.B. Acetone sodium bisulfite, Ascorbinsäure , Ascorbinsäureester, Butylhydroxyanisole (BHA), Butylhydroxytoluen (BHT), Cystein, Nordihydroguaiaretinsäure (NDGA), Monothioglycerol, Sodiumbisulfit, Sodiummetabisulfit, Tocopherole, Glutathion

### 6.4 Konservierungsmittel:

z.B. m-cresol, Chlorocresol, Phenol, Benzylalcohol, Methylparaben, Propylparaben, Butylparaben, Chlorbutanol, Phenylmerçurinitrat, Phenylmercuriacetat, Thimersal, Benzalkoniumchlorid, Benzethoniumchlorid

### 6.5 Cyclodextrine

z.B. Hydroxypropyl-β-cyclodextrin, Sulfobutylethyl-β-cyclodextrin, γ-Cyclodextrin, α-Cyclodextrin

### 6.6 Albumine:

z.B. Humanes Serum Albumin (HSA), Bovines Serum Albumin (BSA)

### 6.7 Polyhydrische Alkohole:

z.B. Glycerol, Ethanol, Mannitol

### 6.8 Salze:

z.B. Acetat-Salze (z.B. Natriumacetat), Magnesiumchlorid, Calciumchlorid, Tromethamine, EDTA (z.B. Na-EDTA)

### 7. Isotonisierunasmittel:

z.B. Natriumchlorid, Kaliumchlorid, Glucose, Glycerin, Dextrose, Sodiumchlorid, Sodiumsulfat

### Beispiel 2: Kristallisation von Erbitux^{™} mit Ammoniumsulfat

500µL Protein (20 mg/mL in 10mM Phosphat, pH 8,0)
400µL Puffer (10mM Phosphat, pH 8,0)
100µL Präzipitans (gesättigte Ammoniumsulfatlösung in 10mM Phosphat, pH 8,0)

Die Zugabe der Präzipitationsreagenzien zu der Proteinlösung erfolgt in Lösung ("batch-Verfahren"). Nach dem Pipetieren soll die Probe durch "Handschütteln" vermischt werden. Das Verfahren kann bei Raumtemperatur oder 4°C durchgeführt werden.

### Beispiel 3: Kristallisation von Erbitux^{™} mit Ethanol

500µL Protein (20 mg/mL in 10mM Citrat, pH 5,5)
400µL Puffer (10mM Citrat, pH 5,5)
100µL Präzipitans (50% (v/v) Ethanol in 10mM Citrat, pH 5,5)
oder
500µL Protein (20 mg/mL in 10mM Citrat, pH 5,5)
500µL Präzipitans (50% (v/v) Ethanol in 10mM Citrat, pH 5,5)

Die Zugabe der Präzipitationsreagenzien zu der Proteinlösung erfolgt in Lösung ("batch-Verfahren"). Nach dem Pipetieren soll die Probe durch "Handschütteln" vermischt werden. Das Verfahren kann bei Raumtemperatur oder 4°C durchgeführt werden.

### Beispiel 4: Präzipitation von Erbitux^{™} mit Ammoniumsulfat

500µL Protein (20 mg/mL in 10mM Phosphat, pH 8,0)
500µL Präzipitans (gesättigte Ammoniumsulfatlösung in 10mM Phosphat, pH 8,0)
oder
200µL Protein (20 mg/mL in 10mM Phosphat, pH 8,0)
800µL Präzipitans (gesättigte Ammoniumsulfatlösung in 10mM Phosphat, pH 8,0)
oder
500µL Protein (20 mg/mL in 10mM Citrat, pH 5,5)
500µL Präzipitans (gesättigte Ammoniumsulfatlösung in 10mM Citrat, pH 5,5)
oder
200µL Protein (20 mg/mL in 10mM Citrat, pH 5,5)
800µL Präzipitans (gesättigte Ammoniumsulfatlösung in 10mM Citrat, pH 5,5)

Die Zugabe der Präzipitationsreagenzien zu der Proteinlösung erfolgt in Lösung ("batch-Verfahren"). Nach dem Pipetieren soll die Probe durch "Handschütteln" vermischt werden. Das Verfahren kann bei Raumtemperatur oder 4°C durchgeführt werden.

### Beispiel 5: Präzipitation von Erbitux^{™} mit PEG

500µL Protein (20 mg/mL in 10mM Phosphat, pH 8,0)
500µL Präzipitans (50% (w/v) PEG 4000 in 10mM Phosphat, pH 8,0)
oder
500µL Protein (20 mg/mL in 10mM Citrat, pH 5,5)
500µL Präzipitans (50% (w/v) PEG 4000 in 10mM Citrat, pH 5,5)
oder
500µL Protein (20 mg/mL in 10mM Phosphat, pH 8,0)
500µL Präzipitans (50% (w/v) PEG 8000 in 10mM Phosphat, pH 8,0)
oder
500µL Protein (20 mg/mL in 10mM Citrat, pH 5,5)
500µL Präzipitans (50% (w/v) PEG 8000 in 10mM Citrat, pH 5,5)

Die Zugabe der Präzipitationsreagenzien zu der Proteinlösung erfolgt in Lösung ("batch-Verfahren"). Nach dem Pipetieren soll die Probe durch "Handschütteln" vermischt werden. Das Verfahren kann bei Raumtemperatur oder 4°C durchgeführt werden.

### Beispiel 6: mikroskopische Untersuchung der Kristallform

Die in den Beispielen 1 und 2 erhaltenen Kristalle wurden mikroskopisch untersucht. Dabei konnte sowohl die für Kristalle typische doppelbrechenden Eigenschaften als auch die für Proteine typische Anfärbbarkeit mit Coomassie Brilliant Blue detektiert werden.
Es wurden Kristalle von ca. 50-200 µm Größe gefunden.

### Beispiel 7: Untersuchung auf Nativität der Präzipitate

Die in den Beispielen 4 und 5 erhaltenen Präzipitate wurden re-dispergiert und FT-IR-spektrometrisch untersucht. Dabei waren die Amid I-2. Ableitung-Spektren des Ausgangsmaterials vor Präzipitation als auch des re-dispergierten Präzipitats kongruent.

### Beispiel 8: Durchführung der in der WO02072636 beschriebenen Methode zur Kristallisation

Zur Überprüfung der Ergebnisse der Patent-Anmeldung WO02072636 wurde in einem Kontrollexperiment, wie in der WO02072636 beschrieben, zuerst versucht, mittels des Wizard I-Screens Kristalle zu erhalten, die anschließend als Seed-Kristall verwendet werden können. Es wurden zwar bei den Präzipitationsbedingungen unter Verwendung von Calciumchlorid oder Calciumacetat nadelförmige Kristalle erhalten, die allerdings ebenso in Citratpufferlösung ohne Protein entstanden. Somit werden mit dem in der W002072636 beschriebenen Verfahren sowohl aus der Proteinlösung als auch aus der Negativkontrolle (ohne Protein) die beschriebenen nadelförmigen Kristalle erhalten werden. Daraus wird deutlich, dass es sich hier vermutlich allenfalls um Proteineinschlüsse in Kristalle des Präzipitionsreagenzes handelt.

## Patentansprüche

1. Verfahren zur Herstellung eines Kristalls von Mab C225 (Cetuximab) oder Mab h425 (EMD72000) und/oder einer ihrer Varianten und/oder Fragmente, welcher durch Auflösen oder Suspendieren in wässrigem Medium zu biologisch aktivem Antikörperprotein führt, **dadurch gekennzeichnet, dass** der in wässriger Lösung gelöste oder suspendierte Antikörper und/oder eine seiner Varianten und/oder Fragmente durch ein Präzipitationsreagenz ausgefällt werden und das Ausfällungsprodukt abgetrennt wird.

2. Verfahren nach Anspruch 1, **dadurch gekennzeichnet, dass** als Präzipitationsreagenz Salze, Polymere und/oder organische Lösungsmittel verwendet werden.

3. Verfahren nach Anspruch 1 oder 2, **dadurch gekennzeichnet, dass** als Präzipitationsreagenz Ammoniumsulfat, Natriumacetat, Natriumcitrat, Kaliumphosphat, PEG und/oder Ethanol verwendet wird.

4. Verfahren nach Anspruch einem oder mehreren der Ansprüche 1 bis 3, **dadurch gekennzeichnet, dass** als Präzipitationsreagenz Ammoniumsulfat, PEG und/oder Ethanol verwendet wird.

5. Verfahren nach einem oder mehreren der Ansprüche 1 bis 4, **dadurch gekennzeichnet, dass** das Verfahren im "batch"- Format durchgeführt wird.

6. Pharmazeutische Zubereitung enthaltend wenigstens ein Kristall von Mab C225 (Cetuximab) oder Mab h425 (EMD72000) und/oder einer ihrer Varianten und/oder Fragmente, hergestellt nach einem Verfahren nach einem oder mehreren der Ansprüche 1 bis 5, sowie gegebenenfalls Träger- und/oder Hilfsstoffe und/oder weitere pharmazeutische Wirkstoffe.

7. Pharmazeutische Zubereitung nach Anspruch 6, **dadurch gekennzeichnet, dass** die Antikörperkonzentration 10 - 200 mglml beträgt.

8. Pharmazeutische Zubereitung nach Anspruch 7, **dadurch gekennzeichnet, dass** die Antikörperkonzentration 50 - 150 mg/ml beträgt.

9. Verwendung eines Kristalls von Mab C225 (Cetuximab) oder Mab h425 (EMD72000) und/oder einer ihrer Varianten und/oder Fragmente, hergestellt nach einem Verfahren gemäß nach einem oder mehreren der Ansprüche 1 bis 5 als lagerstabiles Arzneimittel.

10. Verwendung eines Kristalls von Mab C225 (Cetuximab) oder Mab h425 (EMD72000) und/oder einer ihrer Varianten und/oder Fragmente, hergestellt nach einem Verfahren nach einem oder mehreren der Ansprüche 1 bis 5 zur Herstellung eines Medikaments, welches den biologisch aktiven Antikörper und/oder eine seiner Varianten und/oder Fragmente in präzipitierter nicht-kristalliner, präzipitierter kristalliner oder in gelöster oder suspendierter Form enthält.

11. Verwendung nach Anspruch 10 zur Herstellung eines Medikaments zur Behandlung und/oder Prophylaxe von Tumoren und/oder Tumormetastasen.

12. Verwendung nach Anspruch 11, wobei der Tumor aus der Gruppe ausgewählt ist, bestehend aus Gehirntumor, Tumor des Urogenitaltrakts, Tumor des lymphatischen Systems, Magentumor, Kehlkopftumor, Monozytenleukämie, Lungenadenokarzinom, kleinzelliges Lungenkarzinom, Bauchspeicheldrüsenkrebs, Glioblastom und Brustkarzinom.
